# EUROPÄISCHE PATENTSCHRIFT

(11) **EP 0 578 345 B1**
(45) Veröffentlichungstag und Bekanntmachung des Hinweises auf die Patenterteilung: **22.01.1997**
(21) Anmeldenummer: 93250190.1
(22) Anmeldetag: 25.06.1993
(51) Int. Cl.: A61F 2/34, A61L 27/00

(54) **Hüftpfannenendoprothese**
Acetabular cup endoprosthesis
Endoprothèse de coque acétabulaire

(30) Priorität: 28.06.1992 DE 9208752 U
(43) Veröffentlichungstag der Anmeldung: 12.01.1994
(73) Patentinhaber: ARTOS Medizinische Produkte GmbH, 12277 Berlin (DE)
(72) Erfinder: Buchholz, Gerald, Dipl.-Ing., D-10709 Berlin (DE); Fischer, Hans-Joachim, Dr. Ing., D-12277 Berlin (DE); Kranz, Curt, Dr. Ing., D-10825 Berlin (DE)
(74) Vertreter: Christiansen, Henning, Dipl.-Ing.

(56) Entgegenhaltungen:
- EP-A- 0 237 751
- EP-A- 0 291 562
- EP-A- 0 318 679
- EP-A- 0 358 600
- EP-A- 0 404 680
- EP-A- 0 456 580
- DE-U- 9 208 752
- US-A- 4 650 491

## Beschreibung

Die Erfindung betrifft eine zementfrei zu implantierende Hüftpfannenendoprothese der im Oberbegriff des Anspruchs 1 angegebenen Art.

Eine solche ist beispielsweise aus der EP-A-0 237 751 bekannt.

Bei deren Pfanne sind die beim selbstschneidenden Einschrauben der Pfanne in das Acetabulum in das Knochengewebe einschneidenden, die Gewindegänge bildenden radial nach außen scharfe Schneiden aufweisenden Eingriffselemente (Gewindesegmente) im Querschnitt keilförmig, wobei der Keilwinkel so groß ist, daß durch eine bei der drehenden Einschraubbewegung vorangeführten, auf eine Schneidnut folgende Schneidkante zur weiteren Vertiefung der von dem vorangehenden Gewindesegment vorgeschnittenen Nut ein V-förmiger Span geschnitten werden muß. Dieser Span kann in seinen Querschnittsabmessungen bei tiefer eindringendem Gewindesegment im Bereich der letzten Windungen die volle Flankenbreite überdecken. Es ist ersichtlich, daß damit eine erhebliche Schneidarbeit verbunden ist, die zu einer starken Vergrößerung des Einschraubmoments beiträgt.

Andererseits kann es aber auch vorkommen, daß die Schneidkante wegen der Härte des Knochengewebes im Bereich der Corticalis nicht in der Lage ist, einen vollständigen Span abzuheben. In diesem Fall führt das mit dem fortschreitenden Eindrehen erfolgende tiefere Eindringen der nachfolgenden Gewindesegmente zu einem Aufspalten des Knochens, wie es nur mit dem Spalten von Holz durch eine Axt verglichen werden kann. Bekanntlich können sich derartige durch Keilwirkung an scharf gerundeten Spalten hervorgerufene Risse in Werkstoffen äußerst schnell und weitreichend ausbreiten.

Bei einer weiteren, aus der EP-A-0 358 600 bekannten Schraubpfanne, die eine Weiterentwicklung der aus der EP-A-0 237 751 bekannten Schraubpfanne darstellt, sind die Spitzenbereiche der die Gewindegänge bildenden Gewindesegmente als Flachgewinde in der Weise ausgestaltet, daß die radial nach außen gerichteten Kanten der Gewindegänge flächig stumpf ausgebildet sind und sich die Flanken in diesem Spitzenbereich senkrecht zur Mittelachse der Pfanne erstrecken. Hierbei ist zwar die Möglichkeit der Rißbildung aufgrund der radialen Keilwirkung der Gewindesegmente in den radial außen gelegenen Bereichen der Segmente verringert. Nachteilig hierbei ist dennoch, daß im Bereich des übrigen - nahe der Pfannenoberfläche gelegenen-Grundes der die Gewindegänge bildendenden Segmente aufgrund der dort erheblich von 90° zur Mittelachse abweichenden Flankenwinkel weiterhin eine beträchtliche Keil- und damit die Knochensubstanz gefährdende Spaltwirkung auftritt.

Die aus der EP-A-0 318 679 bekannte Schraubpfanne weist dagegen ein vollständiges Flachgewinde auf, bei dem die Flankenflächen der Gewindegänge genau senkrecht zur Pfannenmittelachse gerichtet sind. Die radial nach außen weisenden Kanten sind ebenfalls flächig stumpf ausgebildet. Damit ist jede das Knochengewebe im Bereich des Gewindes aufkeilende Wirkung verhindert.

Nachteilig bei den vorgenannten Pfannen ist, daß die beim selbstschneidenden Einschrauben ausgeräumten Gewindegänge genau dem Profil der Segmente der Pfanne entsprechen - was sowohl bei den vorgenannten im Querschnitt dreieckigen als auch bei den im Querschnitt rechteckigen Gewindesegmenten der Fall ist, da sie ihr Profil vollständig in den Knochen einschneiden sollen. Daraus folgt aber, daß die Pfannen sich sehr leicht - und im wesentlichen ohne Selbsthemmung - wieder ausdrehen lassen, was im Sinne der zu erzielenden Fixierung unerwünscht ist.

Der Erfindung liegt die Aufgabe zugrunde, die Form und insbesondere die Gewindeform der Hüftpfannenendoprothese der eingangs genannten Gattung derart weiterzubilden, daß die Endoprothese einerseits mit geringen Schneidkräften selbstschneidend in das Acetabulum einzuschrauben ist, andererseits aber auch eine genügende Fixierung zum Erzielen einer ausreichenden Primär- und Langzeitstabilität der eingedrehten Endoprothese gegeben ist.

Diese Aufgabe wird mit den kennzeichnenden Merkmalen des Anspruchs 1 gelöst.

Die Erfindung schließt die Erkenntnis ein, daß bei Gewindesegmenten einer selbstschneidenden Schraubpfanne ein geringer Keilwinkel der Flanken in Bezug auf eine Senkrechte zur Mittelachse der Pfanne aufgrund der Kompressibilität des Knochengewebes nicht zu einer schädlichen Spaltkeil-Wirkung führt, sondern lediglich den Widerstand beim Ein- und - was besonders wichtig ist - beim Ausschrauben heraufsetzt. Der gefundene Winkelbereich führt auch beim vollständigen Eintreiben des letzten mit voller Tiefe in das vorgeschnittene Gewinde eingreifenden Gewindesegments nicht zu einem spaltenden Aufkeilen der geschnittenen Gewindenut im Bereich ihrer spitz- bzw. hier rechtwinkligen Kanten. Statt dessen führt die erzielte Kompression zu einer selbsthemmenden Wirkung, welche einem Wiederausdrehen der Pfanne einen Reibungswiderstand entgegensetzt, der in der Operationsphase bei notwendigen Korrekturen zwar noch ohne weiteres überwunden werden kann, der aber für eine gute Primärfixierung der Pfanne sorgt, so daß bis zum vollständigen Einwachsen durch zusätzliche Kallusbildung ein unbeabsichtigtes Verdrehen sicher verhindert ist.

Durch Versuche wurde gefunden, daß der Winkel zwischen der Schneidflanke und der Mittelachse der Pfanne unter den vorgenannten Bedingungen vorzugsweise zwischen 87° und 89° bzw. zwischen 91° und 93° im Sinne einer Verjüngung des Zahnquerschnitts in radialer Richtung nach außen beträgt.

Damit wird erreicht, daß, bedingt durch die geringfügig angewinkelten Flanken der Gewindesegemente bildenden "Zähne", einerseits im Bereich der äußeren Schneidkanten keine großen axialen Drücke auf den umgebenden Knochen ausgeübt werden und daß andererseits, insbesondere nahe dem Fußbereich der Gewindesegmente, beim Eindrehen lediglich eine vorteilhafte Kompression - ohne Gefahr der Spaltung - des angrenzenden Knochens bewirkt wird. Nach Abschluß des Eindrehvorgangs bleibt damit eine gewisse Kompressionswirkung, über die gesamte Fläche der Gewindeflanken, des Knochengewebes erhalten und führt zur Selbsthemmung der Schraubpfanne, woraus eine verbesserte Primärstabilität der erfindungsgemäßen Schraubpfanne resultiert. Beim weiteren Einheilen erzeugt dann ein Anwachsen der beim selbstschneidenden Einschrauben erzeugten Knochenspäne in den Schneidnuten die notwendige sekundäre oder Langzeit-Fixation. Diese Feststellungen gelten insbesondere für den Bereich der Pfanne, dessen Gewindesegmente ihre endgültige Position im Bereich der Kortikalis des Acetabulums finden. Die Abmessung der stumpfen radial nach außen gerichteten Fläche der das eigentliche Gewinde bildenden, in das Knochengewebe eingreifenden Zahnbereiche beträgt in Richtung der (vertikalen) Mittelachse der Pfanne im wesentlichen zwischen einem Sechstel und geringfügig mehr als der Hälfte der Höhe eines Gewindeganges.

Unter Berücksichtigung des Umstands, daß das Knochengewebe im Bereich der Innenfläche des Acetabulums im Bereich des Pfannenrandes in Form von Kortikalis relativ hart und im Bereich des Pfannendaches in Form von Spongiosa relativ weich ist, konnte die Höhe der Gewindezähne in Richtung eines senkrecht zur Mittelachse der Pfanne weisenden Radius in Richtung auf das Pfannendach zunehmend gewählt werden. Damit wurde eine Außenkontur erzielt, bei der die die maximale Erhebung der die Gewindeflanken bildenden Zähne auf einer Kugelkontur liegen. Die Fußbereiche der Gewindeflanken enden auf der Außenoberfläche der sphärischen Pfannenform, die ebenfalls einer Kugeloberfläche angenähert ist. Die Mittelpunkte beider Kalotten sind aber auf der Mittelachse der Pfannenprothese relativ zueinander versetzt angeordnet. Der Versatz entspricht bevorzugt dem beim Einschrauben der Pfanne zu überwindenden Hub in axialer Richtung. Damit greifen bereits beim Ansetzen der Pfannenprothese zum selbstschneidenden Einschrauben nahezu sämtliche Gewindezähne in das Knochengewebe ein und sorgen somit für eine gute Führung beim Schneiden. Während im Bereich des unteren Pfannenrandes für das Einschneiden des Gewindes eine Mehrzahl von Gewindegängen zur Verfügung steht - was der hier herrschenden Corticalis-Struktur angepaßt ist - erfolgt das Einschneiden des Gewindes im Bereich des Pfannendaches über einen relativ kleinen Drehwinkelbereich. Da die Oberfläche der Pfannenprothese hier in der endgültigen Position in einem Bereich von Spongiosa anliegt, ist die Gefahr eines spaltendenden Aufkeilens geringer. Die erste, nahe dem Pfannendach gelegene Windung des Gewindes kann daher einen dreieckigen spitzwinkligem Querschnitt aufweisen, um ein vollständiges Eindringen der gesamten Segmenttiefe über einen Gewindegang oder sogar nur einen Teil eines Gewindegangs zu ermöglichen.

Insgesamt wird durch die beschriebenen Maßnahmen erreicht, daß die Kräfte während des gesamten Eindrehvorgangs gleichmäßig gering sind und die im Bereich des Pfannendaches gelegenen Gewindegänge zur Erhöhung der Primärstabilität weit in die weiche Spongiosa im Bereich des Pfannendaches eindringen können, wodurch eine hohe Verankerungssicherheit gegeben ist. Die sich entsprechend unterschiedlich weit in radialer Richtung erstreckenden Gewindesegmente machen dabei ein tiefes und damit sicher geführtes Ansetzen der Schraubpfanne möglich, wodurch eine seitliche Führung von Beginn des Eindrehvorgangs an sichergestellt ist. Weiterhin wirken sich Winkelfehleinstellungen beim Einschrauben aufgrund der Gewindegeometrie nicht auf die Verankerungssicherheit aus.

Bei einer vorteilhaften Weiterbildung der erfindungsgemäßen Schraubpfanne ist die an den äußeren Kanten der Schneiden gemessene Gewindezahndicke der Gewindesegmente, die zwischen zwei vollständig ausgebildeten Gewindesegmenten angeordnet sind, konstant. Dadurch tritt die nachteilige Aufkeilwirkung aufgrund eines größeren nachfolgenden Gewindezahns nur kurzzeitig für jeweils nur einen kleinen Bereich des Knochenmaterials während des Eindrehvorgangs auf. Lediglich die vorderen und hinteren Gewindesegmente eines Gewindegangs sind formbedingt etwas kleiner ausgebildet.

Bei einer weiteren vorteilhaften Weiterbildung weist die erfindungsgemäße Schraubpfanne eine vom Pfannenrand zum Pfannendach abnehmende Wanddicke auf. Dadurch wird eine physiologische Krafteinleitung in den Knochen erreicht. Die abnehmende Wanddicke vom Pfannenrand her verhindert Spannungsspitzen am unteren Pfannenbereich und es wird ein gleichmäßiger, physiologischer Spannungsverlauf erzielt. Unphysiologische Knochenumbauvorgänge werden durch die stetige Krafteinleitung verhindert. Die Vermeidung dieser Knochenumbauvorgänge ist eine wichtige Voraussetzung für eine Langzeitstabilität des Implantats.

Die Schraubpfanne ist bei der bevorzugten Ausführungsform mit einem zweigängigen, durch Unterbrechungen in Form von Schneidnuten in Gewindesegmente aufgeteilten, Gewinde versehen, wobei die Gewindesegmente in Schneidrichtung jeweils die Schneidnut begrenzende Span- und Freiwinkel aufweisen. Der Freiwinkel bewirkt, daß die auf die Schneide folgenden Schneidflächen der Gewindesegmente keinen radialen Druck auf das umgebende Knochenmaterial ausüben und ermöglicht zudem ein Ausdrehen der Schraubpfanne im Falle einer notwendigen Revision.

Die erfindungsgemäße Schraubpfanne besteht vorzugsweise aus einer TiAl6V4-Titanlegierung und kann mit einer Hydroxylapatitkeramikbeschichtung versehen sein, um das Anwachsen von Knochenmaterial an die Schraubpfannenoberfläche zu verbessern.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 einen Querschnitt durch eine hälftige Darstellung eines Ausführungsbeispiels der erfindungsgemäßen Hüftpfannenendoprothese entlang der Linie A-A gemäß Figur 2 sowie
Figur 2 eine schematische Draufsicht auf einen drei Gewindesegmente umfassenden Sektor der Pfannenprothese gemäß Figur 1.

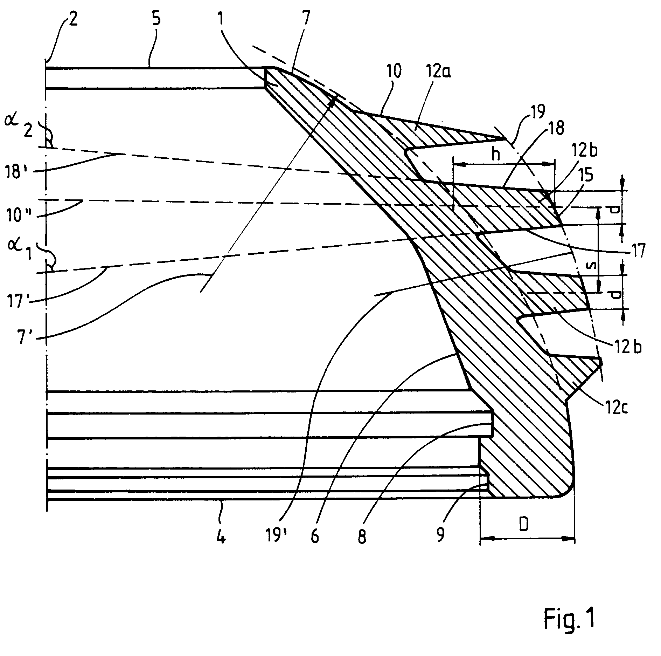

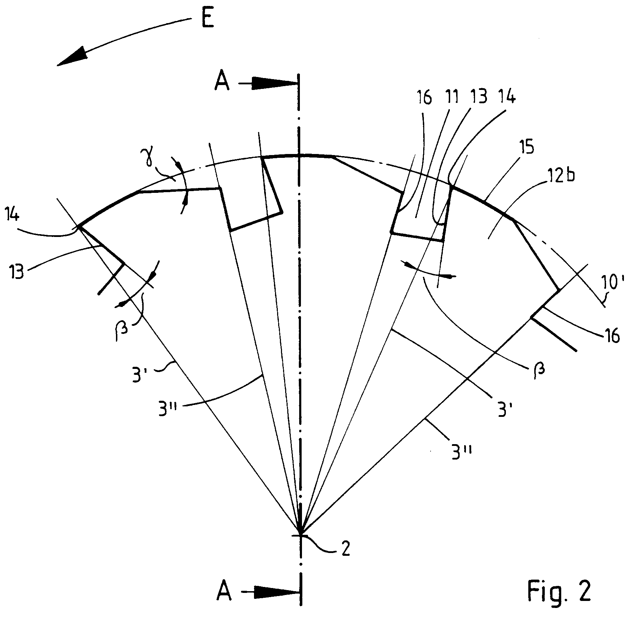

In Figur 1 ist ein axialer Schnitt durch eine hälftige Darstellung eines Ausführungsbeispiels der erfindungsgemäßen Schraubpfanne 1 einer Hüftpfannenendoprothese entlang der Linie A-A gemäß Figur 2 wiedergegeben. Die in der Zeichnung nicht wiedergegebene andere Hälfte der Schraubpfanne ist - wegen der zweigängigen Ausführung des Gewindes - vollständig spiegelbildlich.

Ein hier nicht dargestelltes, mit einem Titandeckel versehenes, Inlay aus Polyethylen wird zur Aufnahme der Gelenkkugel in die äußere Schraubpfanne 1 eingesetzt. Die Innenwandung 6 der äußeren Schraubpfanne 1 weist im Bereich des unteren Randes 4 eine obere und eine untere umlaufende radiale Vertiefung 8 und 9 auf, in die das entsprechend geformte Inlay aufgenommen und gestützt wird. Die radiale Tiefe beider Vertiefungen 8 und 9 nimmt in Richtung auf den unteren Teil 4 der Pfanne ab. Aufgrund dieser verbesserten Stützung wird der Reibschluß und somit auch der Verschleiß des Polyethylen-Inlays vermindert.

Die Wanddicke D der Schraubpfanne 1 nimmt vom Pfannendach 5 ausgehend in Richtung auf den unteren Teil 4 ab. Die Außenwandung 7 der Schraubpfanne 1 weist ein zweigängiges Gewinde 10 mit konstanter Steigung s auf, welches durch Unterbrechungen bildende Schneidnuten 11 in Gewindezähne 12 unterteilt ist.

In der gemäß Figur 1 gewählten Darstellung verläuft der axiale Schnitt durch den vorderen Bereich der Gewindesegmente bildenen Zähne 12a, 12b und 12c. Dabei liegen sowohl die äußeren Schneidkanten 15 als auch die äußeren Kanten 14 der Schneiden 13 (siehe hierzu auch Figur 2) auf einem Kreisbogen 19 mit konstantem Radius 19'. Bei einer gleichbleibenden Gewindezahndicke d der äußeren Schneidkanten 15 der Gewindezähne 12b im mittleren Bereich des Gewindes 10 nimmt die mittlere Höhe h eines Gewindezahns, die dem entlang der Mittelebene 10'' des Gewindeganges 10' gemessenen Abstand zwischen der Außenwandung 7 der Schraubpfanne 1 und der äußersten Erstreckung der Schneidkante 15 der Schneide 13 der Gewindezähne 12b in senkrechter Richtung zur Mittelachse 2 der Pfanne entspricht, in Richtung auf das Pfannendach 5 hin zu. Der obere und der untere Gewindezahn 12a und 12c weisen demgegenüber eine kleinere Zahndicke d auf. Dies ist durch den schraubwendelförmigen Gewindegang 10' bedingt. Insgesamt weichen nur wenige Gewindezähne 12 im vorderen und hinteren Bereich des obersten Gewindegangs 10' von der Form der Gewindezähne mit im wesentlichen konstanter vertikaler Erstreckung, d.h. Dicke d, ab.

Weiterhin weisen der Radius 7' der Außenwandung 7 der Schraubpfanne 1, welcher dem Radius des vorbereiteten Acetabulum entspricht, und der Radius 19' des Kreisbogens 19, auf dem die äußeren Schneidkanten 15 der Gewindezähne 12a, 12b und 12c und die äußeren Kanten 14 der Schneiden 13 gelegen sind, unterschiedliche Mittelpunkte auf. Diese sind in Richtung der Mittelachse versetzt. Dadurch wird auf vorteilhafte Weise nicht nur die Gewindezahnhöhe h, sondern auch die realisierbare Eindringtiefe der Gewindezähne 12 im unteren Pfannenbereich 4 vergrößert.

Ausgehend von den äußeren Schneidkanten 15 verlaufen die Flanken 17 und 18 der Gewindezähne 12 derart, daß die von den Flanken 17 und 18 auf die Schraubpfannenmittelachse 2 projizierten Linien 17' und 18' die Schraubpfannenmittelachse 2 unter einem Winkel α₁ und α₂ von 87° bzw. 93° schneiden. Dies bedeutet, daß die Flanken 17 und 18 jeweils eine Linie, die rechtwinklig zur Schraubenpfannenmittelachse 2 gerichtet ist und durch den Schnittpunkt der Flanke 17 bzw. 18 und äußeren Schneidkante 15 verläuft, mit einem Winkel von jeweils im wesentlichen 3° schneiden. Bei anderen nicht dargestellten Ausführungsbeispielen kann dieser Winkelbereich von 1° und bis zu 3° reichen.

Anhand von Figur 2 soll im folgenden die Form der Gewindezähne 12b im mittleren Bereich des Gewindeganges 10' anhand einer Draufsicht erläutert werden. Die Gewindezähne 12b weisen jeweils eine schneide 13 auf, die einen Spanwinkel β von vorzugsweise 5° zu einer durch die Schraubpfannenmittelachse 2 gelegte Radialebene 3' aufweist. Die äußeren Schneidkanten 15 liegen zwischen den Schneiden 13 und den Abschlußkanten 16 der Gewindezähne 12b, wobei die äußeren Schneidkanten 15 im - bezogen auf die Einschraubrichtung E - vorderen (Schneid-)Bereich der Gewindezähne 12b auf dem wendelförmigen Gewindegang 10' des Gewindes 10 liegen und im hinteren Bereich unter einem Freiwinkel γ von vorzugsweise 30° zur rückwärtigen Kante 16 hin verjüngen. Die rückwärtigen Kanten 16 fallen vorzugsweise in eine die Pfannenmittelachse 2 einschließende Radialebene 3''. Die rückwärtigen Kanten 16 und eine in Einschraubrichtung E nach einer Unterbrechung 11 folgende Schneide 13 des jeweils nachfolgenden Zahns weisen vorzugsweise einen relativen Winkel von 5° auf, wodurch sich der radiale Abstand der Abschlußkante 16 zur Schneide 13 in radialer Richtung verkleinert.

Es ist ersichtlich, daß, wenn die in Figur 1 dargestellte Pfanne zum selbstschneidenden Einschrauben an die Innenseite des sphärisch vorbereiteten Acetabulums (repräsentiert durch den Kreisbogen 19) angesetzt wird, sämtliche mit ihren radial nach außen weisenden tangential gerichteten Flächen der - Gewindesegmente bildenden - Zähne 12a bis 12d des Gewindes, die durch den Kreisbogen 19 definierte sphärische Fläche gemeinsam tangieren. Beim Drehen in Einschraubrichtung greifen die die Schneidnuten begrenzenden Schneidkanten in das Knochengewebe ein und erzeugen eine schraubenförmige Einrillung, in der die nachfolgenden Gewindesegmente zum tieferen Einschneiden folgen. Die jeweils vorangeführten Gewindesegmente folgen einander profilgetreu in der geschnittenen Nut, wobei ein Ausräumen des Knochens im äußersten Bereich nahe der radial außen gelegenen Schneide erfolgt. Der hier ausgeräumte Span hat einen Querschnitt, dessen radial außen gelegene Ecken Winkel aufweisen, die größer als 90° sind. Die weiter innen gelegenen Schneidkanten des Schneidenbereiches der Gewindesegmente schneiden sich jedoch nicht in den Knochen ein, sondern komprimieren ihn geringfügig, soweit es den dem unteren Pfannenrand benachbarten Kortikalisbereich betrifft. Die Gewindesegmente dringen dabei - sich in tangentialer Richtung fortbewegend - fortlaufend tiefer in den Knochen ein. Die dabei erzeugte Keilwirkung trägt wegen der geringen Steigung des Keils von zusammen bis zu sechs Grad nicht zu einer Spaltung des Knochens, sondern lediglich zu seiner Kompression bei, welche aufgrund der Reibungsverhältnisse (Metall auf glattem feuchten Knochenmaterial) zur Selbsthemmung des so gebildeten Keils - und somit auch des gesamten so gebildeten Gewindes - führt.

Der breite, stumpfe radial gerichtete Bereich der Gewindesegmente trägt ebenfalls dazu bei, daß eine Spaltung des Knochengewebes vermieden ist. Nur die oberste für den Spongiosabereich bestimmte Gewindewindung weicht in ihrer Dimensionierung ab, da sie mit dreieckigem Querschnitt unter Führung der übrigen Windungen bei dem weichen, spongiösen Knochenmaterial über eine relativ kurze Distanz die volle Eindringtiefe erreichen kann. Hier besteht eine Gefahr der Rißbildung durch Spaltung nicht, da die entstehenden Spannungen vom Knochengewebe selbst lokal abgebaut werden können.

Die Erfindung beschränkt sich in ihrer Ausführung nicht auf das vorstehend angegebene bevorzugte Ausführungsbeispiel.

## Patentansprüche

1. Zementfreie Hüftpfannenendoprothese mit einer konvexen äußeren Schraubpfanne (1), die an ihrer Außenwandung (7) ein selbstschneidendes Gewinde (10) konstanter Steigung mit spiralwendelförmigem Gewindegang und Schneidnuten aufweist, wobei mindestens ein Teil (12b) der sich in radialer Richtung nach außen verjüngenden und Flanken der Gewindesegmente bildenden vorspringenden Eingriffsbereiche (12a, 12b, 12c) des Gewindes, ausgehend von den äußeren Schneidkanten (15), durchgehend einen von 90° abweichenden Winkel zur Mittelachse (2) der Pfanne aufweist,
**dadurch gekennzeichnet,**
daß die Flanken (17, 18) von Gewindesegmenten (12b) an ihrer proximalen Oberseite mit der Mittelachse einen Winkel von 91°≦ α₂ ≦ 93° und an ihrer distalen Unterseite einen Winkel von 87° ≦ α₁ ≦ 89° bilden und
daß die besagten Gewindesegmente (12b) in ihren einen maximalen radialen Abstand von der Mittelachse aufweisenden Bereichen stumpf in einer sich im wesentlichen auf einer Kugelkontur (19) erstreckenden Fläche enden, deren Erstreckung (d) parallel zur Mittelachse (2) mindestens einem Sechstel der Höhe eines Gewindeganges entspricht.

2. Hüftpfannenendoprothese nach Anspruch 1, **dadurch gekennzeichnet,** daß die mittlere Höhe (h) der Gewindesegemente, die deren maximaler radialer Erstreckung über den Pfannengrund in einer Richtung senkrecht zur Mittelachse der Schraubpfanne (1) entspricht, in Richtung zum Pfannendach (5) hin zunimmt.

3. Hüftpfannenendoprothese nach einem der vorangehenden Ansprüche, **dadurch gekennzeichnet,** daß die äußeren Kanten (14) der Schneiden (13) der Gewindesegmente (12a, 12b, 12c) in einer die Mittelachse der Pfanne einschließenden Ebene auf einem Kreisbogen (19) gelegen sind, dessen Mittelpunkt sich auf der Pfannenmittelachse befindet.

4. Hüftpfannenendoprothese nach Anspruch 3, **dadurch gekennzeichnet,** daß der Radius (19') des im axialen Schnitt die Kugelkontur repräsentierenden Kreisbogens (19) auf dem die äußeren Kanten (14) der Schneiden (13) der Gewindesegmente gelegen sind, gegenüber dem Mittelpunkt des Radius (7') ihrer die Gewindesegmente tragenden Außenwandung (7) auf der Mittelachse der Pfanne dem Pfannendach näher gelegen ist.

5. Hüftpfannenendoprothese nach Anspruch 4, **dadurch gekennzeichnet,** daß die Länge beider Radien (19', 7') übereinstimmt.

6. Hüftpfannenendoprothese nach einem der Ansprüche 4 oder 5, **dadurch gekennzeichnet,** daß der Abstand der Mittelpunkte des Kreisbogens (19) und der Außenwandung (7) dem Hub der Pfanne (1) beim Einschrauben in das Acetabulum zwischen erstem Kontakt zwischen Knochenoberfläche und Gewindesegmenten bis zur vollständig eingeschraubten Position entspricht.

7. Hüftpfannenendoprothese nach einem der vorangehenden Ansprüche, **dadurch gekennzeichnet,** daß die Dicke (d) der besagten Gewindesegmente (12b) an den äußeren Kanten (14) ihrer Schneiden (13) im wesentlichen konstant ist und insbesondere der Hälfte der Höhe eines Gewindeganges entspricht oder geringfügig kleiner ist.

8. Hüftpfannenendoprothese nach einem der vorangehenden Ansprüche, **dadurch gekennzeichnet,** daß die Schneiden (13) der Gewindesegmente (12a, 12b, 12c) einen Spanwinkel (β) von vorzugsweise 5° zu einer durch die Schraubpfannenmittelachse (2) gelegten Radialebene (3') aufweisen.

9. Hüftpfannenendoprothese nach einem der vorangehenden Ansprüche, **dadurch gekennzeichnet,** daß die in Einschraubrichtung hinter den Schneiden (13) liegenden äußeren Schneidkanten (15) der Gewindesegmente (12a, 12b, 12c) mindestens teilweise auf dem wendelförmigen Gewindegang (10') gelegen sind.

10. Hüftpfannenendoprothese nach einem der vorangehenden Ansprüche, **dadurch gekennzeichnet,** daß die Gewindesegmente (12a, 12b, 12c) jeweils eine in einer durch die Schraubpfannenmittelachse (2) gelegten Radialebene (3'') verlaufende und in Eindrehrichtung hinter der Schneide (13) liegende rückwärtige Kante (16) aufweisen.

11. Hüftpfannenendoprothese nach Anspruch 10, **dadurch gekennzeichnet,** daß die äußeren Schneidkanten (15) der Gewindesegmente (12a, 12b, 12c) mindestens in einem Teilbereich nahe der rückwärtigen Kante (16) einen Freiwinkel (γ) von im wesentlichen 30° zum Gewindegang (10') aufweisen.

12. Hüftpfannenendoprothese nach einem der vorangehenden Ansprüche, **dadurch gekennzeichnet,** daß vom Rand (4) ausgehend die Dicke (D) der Gewindesegmente in Richtung zum Pfannendach hin (5) abnimmt.

13. Hüftpfannenendoprothese nach einem der vorangehenden Ansprüche, **dadurch gekennzeichnet,** daß die Innenwandung (6) im Bereich des Rands (4) zwei rechtwinklig zur Pfannenmittelachse (2) umlaufende radiale Vertiefungen (8 und 9) aufweist.

14. Hüftpfannenendoprothese nach Anspruch 13, **dadurch gekennzeichnet,** daß die radiale Tiefe der Vertiefungen (8 und 9) in Richtung zum Pfannendach (5) hin abnimmt.

15. Hüftpfannenendoprothese nach einem der vorangehenden Ansprüche, **gekennzeichnet durch** die Legierung TiAl6V4 als Herstellungswerkstoff.

16. Hüftpfannenendoprothese nach einem der vorangehenden Ansprüche, **gekennzeichnet durch** eine Beschichtung aus Hydroxylapatit-Keramik.

## Claims

1. A cement-free acetabular cup endoprosthesis with a convex, outer screw cup (1) which on its outer wall (7) has self-tapping threading (10) of constant pitch comprising a spiral-shaped thread and cutting grooves, where at least a part (12b) of the projecting engagement zones (12a, 12b, 12c) of the thread, which taper in the radially outward direction and form flanks of the thread segments, commencing from the outer cutting edges (15), continuously form an angle differing from 90° relative to the central axis (2) of the cup, characterised in that on their proximal upper side the flanks (17, 18) of thread segments (12b) form an angle of 91° ≤ α₂ ≤ 93° relative to the central axis and on their distal underside form an angle of 87° ≤ α₁ ≤ 89° and that in their zones which are a maximum radial distance from the central axis the said thread segments (12b) are truncated in a surface which extends substantially on a spherical contour (19) and whose extent (d) parallel to the central axis (2) corresponds to at least one sixth of the height of a thread.

2. An acetabular cup endoprosthesis according to Claim 1, characterised in that the average height (h) of the thread segments, which corresponds to the maximum radial extent of said segments over the base of the cup in a direction at right angles to the central axis of the screw cup (1), increases towards the roof (5) of the cup.

3. An acetabular cup endoprosthesis according to one of the preceding claims, characterised in that the outer edges (14) of the cutting edges (13) of the thread segments (12a, 12b, 12c) lie in a plane, enclosing the central axis of the cup, on a circular arc (19) whose central point lies on the central axis of the cup.

4. An acetabular cup endoprosthesis according to Claim 3, characterised in that the radius (19') of the circular arc (19), which in an axial section represents a spherical contour and on which the outer edges (14) of the cutting edges (13) of the thread segments are located, is situated on the central axis of the cup closer to the roof of the cup compared to the central point of the radius (7') of the outer wall (7), which bears the thread segments.

5. An acetabular cup endoprosthesis according to Claim 4, characterised in that the lengths of the two radii (19', 7') are identical.

6. An acetabular cup endoprosthesis according to one of Claims 4 or 5, characterised in that the distance between the central points of the circular arc (19) and the outer wall (7) corresponds to the movement of the cup (1), upon screwing into the acetabulum, from the first contact between the bone surface and the thread segments up to the fully screwed-in position.

7. An acetabular cup endoprosthesis according to one of the preceding claims, characterised in that the thickness (d) of the said thread segments (12b) at the outer edges (14) of their cutting edges (13) is substantially constant and in particular corresponds to, or is slightly less than, half the height of a thread.

8. An acetabular cup endoprosthesis according to one of the preceding claims, characterised in that the cutting edges (13) of the thread segments (12a, 12b, 12c) form a cutting angle (β) of preferably 5° relative to a radial plane (3') passing through the acetabular cup central axis (2).

9. An acetabular cup endoprosthesis according to one of the preceding claims, characterised in that the outer cutting edges (15) of the thread segments (12a, 12b, 12c), which outer cutting edges are disposed behind the cutting edges (13) in the screwing-in direction, lie at least partially on the spiral-shaped thread (10').

10. An acetabular cup endoprosthesis according to one of the preceding claims, characterised in that the thread segments (12a, 12b, 12c) in each case possess a rear edge (16) which extends in a radial plane (3'') passing through the acetabular cup central axis (2) and is disposed behind the cutting edge (13) in the tapping direction.

11. An acetabular cup endoprosthesis according to Claim 10, characterised in that, at least in a sub-zone close to the rear edge (16), the outer cutting edges (15) of the thread segments (12a, 12b, 12c) form a clearance angle (γ) of substantially 30° relative to the thread (10').

12. An acetabular cup endoprosthesis according to one of the preceding claims, characterised in that, commencing from the edge (4), the thickness (D) of the thread segments reduces towards the roof (5) of the cup.

13. An acetabular cup endoprosthesis according to one of the preceding claims, characterised in that in the region of the edge (4) the inner wall (6) comprises two radial recesses (8 and 9) extending circumferentially at right angles to the cup central axis (2).

14. An acetabular cup endoprosthesis according to Claim 13, characterised in that the radial depth of the recesses (8 and 9) reduces towards the roof (5) of the cup.

15. An acetabular cup endoprosthesis according to one of the preceding claims, characterised by the alloy TiAl6V4 as the manufacturing material.

16. An acetabular cup endoprosthesis according to one of the preceding claims, characterised by a coating of hydroxyl apatite ceramic.

## Revendications

1. Endoprothèse de coque acétabulaire exempte de ciment, comprenant une coque extérieure convexe à visser (1), qui présente sur sa paroi extérieure (7) un pas de vis (10) à auto-découpe avec pente constante doté d'un pas de vis de forme hélicoïdale et de gorges de découpe, dans laquelle une partie (12b) au moins des régions d'engagement (12a, 12b, 12c) du pas de vis, qui se projettent en direction radiale en se rétrécissant vers l'extérieur et qui forment des flancs des segments de filetage, présente, en partant des arêtes de découpe extérieures (15), de façon continue un angle qui diffère de 90° vis-à-vis de l'axe médian (2) de la coque, caractérisée en ce que les flancs (17, 18) des segments de filetage (12b) forment à leur côté proximal supérieur un angle de 91° ≤ α2 ≤ 93° avec l'axe médian et au niveau de leur côté inférieur distal un angle de 87° ≤ α1 ≤ 89°, et en ce que lesdits segments de filetage (12b) se terminent dans leur région, qui présente une distance radiale maximum de l'axe médian, de façon émoussée dans une surface qui s'étend sensiblement sur un contour sphérique (19), dont l'extension (d) parallèlement à l'axe médian (2) correspond à au moins un sixième de la hauteur d'un pas de vis.

2. Endoprothèse de coque acétabulaire selon la revendication 1, caractérisée en ce que la hauteur moyenne (h) des segments de filetage, qui correspond à leur extension radiale maximum au-dessus du fond de la coque dans une direction perpendiculaire à l'axe médian de la coque à visser (1), augmente en direction du sommet (5) de la coque.

3. Endoprothèse de coque acétabulaire selon l'une des revendications précédentes, caractérisée en ce que les arêtes extérieures (14) des tranchants (13) des segments de filetage (12a, 12b, 12c) sont disposées dans un plan qui contient l'axe médian de la coque sur un arc de cercle (19) dont le centre est situé sur l'axe médian de la coque.

4. Endoprothèse de coque acétabulaire selon la revendication 3, caractérisée en ce que le rayon (19') de l'arc de cercle (19) qui représente en coupe axiale le contour sphérique et sur lequel sont disposées les arêtes extérieures (14) des tranchants (13) des segments de filetage, est situé plus proche du sommet de la coque sur l'axe médian de celle-ci, par rapport au centre du rayon (7') de sa paroi extérieure (7) qui porte les segments de filetage.

5. Endoprothèse de coque acétabulaire selon la revendication 4, caractérisée en ce que la longueur des deux rayons (19', 7') coïncide.

6. Endoprothèse de coque acétabulaire selon l'une ou l'autre des revendications 4 et 5, caractérisée en ce que la distance des centres de l'arc de cercle (19) et de la paroi extérieure (7) correspond à la course de la coque (1) lors du vissage dans l'acétabule depuis un premier contact entre la surface de l'os et les segments de filetage jusqu'à la position totalement vissée.

7. Endoprothèse de coque acétabulaire selon l'une des revendications précédentes, caractérisée en ce que l'épaisseur (d) desdits segments de filetage (12b) est sensiblement constante au niveau des arêtes extérieures (14) de leurs tranchants (13), et correspond en particulier à la moitié de la hauteur d'un pas de vis, ou bien est légèrement inférieure à cette valeur.

8. Endoprothèse de coque acétabulaire selon l'une des revendications précédentes, caractérisée en ce que les tranchants (13) des segments de filetage (12a, 12b, 12c) présentent un angle de découpe (β) de préférence égal à 5° par rapport à un plan radial (3') qui passe à travers l'axe médian (2) de la coque vissée.

9. Endoprothèse de coque acétabulaire selon l'une des revendications précédentes, caractérisée en ce que les arêtes de découpe extérieures (15) situées derrière les tranchants (13) en direction de vissage, des segments de filetage (12a, 12b, 12c), sont disposées au moins partiellement sur le pas de vis hélicoïdal (10').

10. Endoprothèse de coque acétabulaire selon l'une des revendications précédentes, caractérisée en ce que les segments de filetage (12a, 12b, 12c) présentent chacun une arête postérieure (16) qui s'étend dans un plan radial (3'') qui traverse l'axe médian (2) de la coque vissée, et disposée en arrière du tranchant (13) dans la direction de vissage.

11. Endoprothèse de coque acétabulaire selon la revendication 10, caractérisée en ce que les arêtes de découpe extérieures (15) des segments de filetage (12a, 12b, 12c) présentent dans une région partielle proche de l'arête postérieure (16) un angle de dépouille (γ) sensiblement égal à 30° par rapport au pas de vis (10').

12. Endoprothèse de coque acétabulaire selon l'une des revendications précédentes, caractérisée en ce que l'épaisseur (D) des segments de filetage diminue depuis la bordure (4) en direction du sommet de la coque (5).

13. Endoprothèse de coque acétabulaire selon l'une des revendications précédentes, caractérisée en ce que la paroi intérieure (6) présente dans la région de la bordure (4) deux renfoncements (8 et 9) radiaux périphériques perpendiculairement à l'axe médian (2) de la coque.

14. Endoprothèse de coque acétabulaire selon la revendication 13, caractérisée en ce que la profondeur radiale des renfoncements (8 et 9) diminue en direction du sommet (5) de la coque.

15. Endoprothèse de coque acétabulaire selon l'une des revendications précédentes, caractérisée en ce que le matériau de fabrication est de l'alliage TiAl6V4.

16. Endoprothèse de coque acétabulaire selon l'une des revendications précédentes, caractérisée par un revêtement de céramique hydroxylapatite.
